# EUROPÄISCHE PATENTSCHRIFT

(11) **EP 0 792 188 B1**
(45) Veröffentlichungstag und Bekanntmachung des Hinweises auf die Patenterteilung: **23.02.2000**
(21) Anmeldenummer: 95936946.3
(22) Anmeldetag: 17.11.1995
(51) Int. Cl.: B01J 2/16, F26B 3/08

(54) **VERFAHREN UND VORRICHTUNG ZUR TROCKNUNG VON BLUTPLASMA**
METHOD OF AND APPARATUS FOR DRYING BLOOD PLASMA
PROCEDE DE ET APPAREIL POUR SECHAGE DE PLASMA SANGUIN

(30) Priorität: 18.11.1994 DE 4441167
(43) Veröffentlichungstag der Anmeldung: 03.09.1997
(73) Patentinhaber: FRAUNHOFER-GESELLSCHAFT ZUR FÖRDERUNG DER ANGEWANDTEN FORSCHUNG E.V., 80636 München (DE); GLATT GMBH, 79589 Binzen (DE)
(72) Erfinder: LUCK, Thomas, D-80992 München (DE); PRASCH, Armin, D-81371 München (DE); MÄURER, Andreas, D-80634 München (DE); NOWAK, Reinhard, D-79589 Binzen (DE); LUY, Bernard, D-79102 Freiburg (DE)
(74) Vertreter: Pfenning, Meinig & Partner
(86) Internationale Anmeldenummer: DE9501619
(87) Internationale Veröffentlichungsnummer: WO9615849

(56) Entgegenhaltungen:
- EP-A- 0 015 055
- EP-A- 0 403 820
- EP-A- 0 580 176
- CH-A- 646 729
- DE-A- 3 535 536

## Beschreibung

Die Erfindung betrifft ein Verfahren zur Trocknung von Blutplasma, Blutplasmafraktionen bzw. von daraus gewonnenen Produkten mittels eines Wirbelschichtverfahrens und eine entsprechende Vorrichtung zur Durchführung des Verfahrens.

Es ist bekannt, daß die Behandlung von Patienten mit Produkten (Proteine, Proteinfraktionen) aus humanem Blutplasma mit einem sehr hohen Risiko der Krankheitsübertragung durch Viren, z.B. Retroviren oder Hepatitis-Viren, verbunden ist. Vor allem bei Patienten, die an Hämophilie leiden, ist ein sehr hohes Infektionsrisiko vorhanden. Es sind deshalb im Stand der Technik verschiedene Verfahren zur Viren-Inaktivierung bzw. -Eliminierung beschrieben worden. Alle diese Verfahren beeinflussen jedoch die biologische Aktivität des Blutplasmaproduktes.

Die meisten Verfahren schlagen eine Hitze-Inaktivierung vor. So wird z.B. beschrieben ("Le fractionnement plasmatique. Progrès, Problémes et perspectives". Burnouf, T; Ann. pharmaceutiques francaises; 1994, 52, n°3, p. 124-136), eine Hitze-Inaktivierung von flüssigen (bei 60°C über zehn Stunden) und lyophilisierten Plasmaprodukten (Stickstoffatmosphäre, 60°C über zehn Stunden) durchzuführen.

Aus der EP 0 094 611 ist bekannt, daß sich trockene, z.B. lyophilisierte Plasmaproteine mit geringem Verlust an Aktivität pasteurisieren lassen. Ein Kennzeichen derartiger Trockenerhitzungsverfahren ist jedoch die Tatsache, daß vor allem bei größeren Mengen die Wärmeübertragung in solchen Proben sehr schlecht und ungleichmäßig erfolgt, woraus einerseits partielle Überhitzungen und andererseits partiell ungenügende Erhitzungen resultieren. Dies führt einerseits zu unerwünscht hohen Schädigungen des biologischen Wirkstoffes und andererseits dazu, daß die vollständige Virus-Inaktivierung nicht sicher gewährleistet ist.

Aus dem Stand der Technik sind auch chemische Verfahren zur Inaktivierung von Viren bekannt. Zu nennen ist hier z.B. das Solvent/Detergent-Verfahren zur Inaktivierung von Viren, die mit einer Lipidhülle umhüllt sind unter Einsatz von Tri-n-butyl-phosphat unter Zusatz von weiteren Detergenzien, wie z.B. Tween 80®. Ein anderes Verfahren auf chemischer Basis beschreibt die Inaktivierung durch alkylierende Substanzen, wie β-Propiolacton, in Kombination mit einer UV-Behandlung (Bundesanzeiger Nr. 161, 1994, "Bekanntmachungen über Maßnahmen zur Abwehr von Arzneimittelrisiken - Verhinderung des Risikos der Übertragung von hämatogenen Viren bei Arzneimitteln, die durch Fraktionierung aus Plasma humanen Ursprungs hergestellt werden").

Die vorstehend beschriebenen Verfahren haben somit sämtlich den Nachteil, daß die biologische Aktivität der Blutplasmaprodukte negativ beeinflußt wird.

Es hat deshalb nicht an Versuchen gefehlt, hier durch sogenannte "schonende Trocknungsverfahren" Abhilfe zu schaffen. Derartige Verfahren sind die Lyophilisation und Vakuumtrocknungsverfahren. Diese Verfahren haben zwar den Vorteil einer schonenden Produktbehandlung, jedoch ist hier der Nachteil vorhanden, daß diese verfahrenstechnisch sehr aufwendig sind und somit hohe Investitions- und Betriebskosten resultieren. Ungünstig wirkt sich bei diesen Verfahren weiterhin der geringe Durchsatz und die unflexible Prozeßführung aus.

Ausgehend hiervon, ist es deshalb die Aufgabe der vorliegenden Erfindung, ein Verfahren zur schonenden Trocknung von Blutplasma, Blutplasmafraktionen bzw. daraus gewonnenen Blutplasmaprodukten vorzuschlagen, bei dem keine oder nur eine minimale Beeinflussung der biologischen Aktivität des Blutplasmaproduktes vorliegt. Gleichzeitig soll dieses Verfahren einfach und kostengünstig durchführbar sein.

Die Erfindung wird durch die kennzeichnenden Merkmale des Patentanspruches 1 gelöst, vorrichtungsgemäß durch die kennzeichnenden Merkmale des Anspruches 9. Die Unteransprüche zeigen vorteilhafte Weiterbildungen auf.

Erfindungsgemäß wird somit vorgeschlagen, die Blutplasmaprodukte in einer Wirbelschichtkammer zu trocknen. Es ist dazu vorgesehen, daß das zu behandelnde Blutplasmaprodukt in flüssiger Form in einen evakuierbaren Behälter eingesprüht (gemäß der EP-A-0 194 266 B1) wird und mittels eines Fluidisationsgases eine Trocknung im Wirbelbett erfolgt. Die Flüssigkeit kann dabei entweder im Gegenstrom zum Fluidisationsgas geführt sein (Top-Spray-Verfahren) oder im Gleichstrom (Bottom-Spray-Verfahren). Dadurch, daß das flüssige Behandlungsgut in dem evakuierbaren Behälter durch eine geeignete Düse fein verteilt wird, kann durch das Fluidisationsgas eine optimale Behandlung erreicht werden. Die Aufgabe des Fluidisationsgases ist somit zum einen die Verwirbelung des zu behandelnden Gutes und zum anderen die konvektive Wärmeübertragung. Erfindungsgemäß wird deshalb ein erwärmtes Gas als Fluidisationsgas eingesetzt. Durch eine Messung der Produkttemperatur während des Wirbelschichtprozesses und einer darauf basierenden Prozeßsteuerung kann eine produktschonende Trocknung eingehalten werden. Die Temperatur des Fluidisationsgases ist dabei selbstverständlich nach dem zu behandelnden Gut ausgewählt. Die Temperaturauswahl wird so vorgenommen, daß eine möglichst schonende Trocknung herbeigeführt wird. Die Temperatur kann dabei im Bereich von 15 bis 75°C je nach ausgewähltem Behandlungsgut liegen. Als Fluidisationsgas kann entweder Luft oder ein an und für sich aus dem Stand der Technik bekanntes Inertgas, wie z.B. Stickstoff, eingesetzt werden. Die Trocknung wird dabei so lange fortgeführt, bis das Behandlungsgut in feinverteilter Granulatform vorliegt. Die bei diesem erfindungsgemäßen Verfahren erzeugten Granulate weisen hierbei eine Größe von 100 - 200 µm auf. Damit wird deutlich, daß das zu behandelnde Gut in sehr fein verteilter Form vorliegt, so daß hiermit eine optimale Wärmeübertragung vom Fluidisationsgas zum behandelnden Gut gewährleistet ist.

Bevorzugt wird das Verfahren so ausgeführt, daß das Fluidisationsgas im Kreislauf durch den evakuierbaren Behälter geführt wird. In diesem Fall hat somit das Fluidisationsgas gleichzeitig noch die Aufgabe, den Abtransport der verdampften bzw. getrockneten Flüssigkeit vom feuchten Produkt zu gewährleisten. Dazu ist erfindungsgemäß weiter vorgesehen, daß im Kreislauf zur Konditionierung des Fluidisationsgases ein Kondensator (zur Entfeuchtung) und ein Wärmeaustauscher (zur Erwärmung) vorgesehen sind.

Um besonders schonende Trocknungsbedingungen einhalten zu können, kann die Trocknung auch bei reduziertem Betriebsdruck erfolgen. Der Druck kann in diesem Fall bis auf < 500 mbar gesenkt werden. Um den Nachteil der geringeren konvektiven Trocknungsleistung bei reduziertem Betriebsdruck ausgleichen zu können, kann über eine zusätzliche Energiequelle, deren Übertragungsmechanismus nicht an die konvektive Wärmeübertragung gebunden ist, Energie, die zum Trocknen zur Verfügung steht, zugeführt werden. Dies kann z.B. durch Mikrowellenstrahlung, die in das Wirbelbett eingekoppelt wird, erfolgen. Dadurch ergeben sich zusätzliche Freiheitsgrade bei der Steuerung und Durchführung des Trocknungsprozesses und der gezielten Erwärmung.

Erfindungsgemäß wird weiter vorgeschlagen, daß nach dem Ende der Trocknung des zu behandelnden Gutes zusätzlich eine gezielte Wärmebehandlung zur Inaktivierung von Viren eingesetzt wird. Eine derartige Wärmebehandlung wird erst nach nahezu abgeschlossener Trocknung angewendet, da das getrocknete Behandlungsgut in Form des vorstehend beschriebenen feinen Granulates wesentlich wärmestabiler ist als das am Prozeßanfang eingesetzte flüssige Behandlungsgut. Erfindungsgemäß wird deshalb die Wärmebehandlung erst nach der Trocknung angewendet. Es ist jedoch auch möglich, daß die Wärmebehandlung schon zu Beginn der letzten Phase des Trocknungsprozesses eingesetzt wird. Voraussetzung ist jedoch in allen Fällen, daß das zu behandelnde Gut bereits in feinverteilter Granulatform vorliegt. Zur Wärmebehandlung können die üblicherweise zur Inaktivierung von Viren bei trockenen Blutplasmaprodukten bisher schon bekannten notwendigen Betriebsbedingungen eingestellt werden. Wesentlich ist, daß die Inaktivierung in fluidisiertem Zustand erfolgt. Dadurch werden ideale Wärmeübergangsverhältnisse geschaffen, so daß eine gleichmäßige Temperaturbeaufschlagung und damit eine gleichmäßige Inaktivierung erfolgt. Die Inaktivierung kann in Luft, in einer Inertgas- oder in einer mit Ozon angereicherten Luftatmosphäre erfolgen.

Zusätzlich zur konvektiven Energiezufuhr durch das Fluidisationsmedium kann auch noch durch eine externe Energiequelle gezielt Wärme im Produkt erzeugt werden. Vorteilhafterweise wird dies mittels Mikrowellenstrahlung erreicht.

Zusätzlich kann über ein am Umfang des Reaktors im Bereich des Wirbelbettes angebrachtes Fenster aus UV-durchlässigem Material das fluidisierte Produkt mit UV-Licht bestrahlt werden, um eine Inaktivierung von Viren zu erreichen. Gleichzeitig kann auch noch durch einen im Reaktor angebrachten UV-Strahler das Produkt direkt bestrahlt werden.

Das erfindungsgemäße Verfahren erlaubt auch eine chemische Inaktivierung. Eine chemische Inaktivierung der Viren ist dabei nach der Solvent/Detergent-Methode gegeben - in der Weise, daß nämlich dem flüssigen Behandlungsgut, bevor es in die evakuierbare Kammer gesprüht wird, bereits ein entsprechendes Lösungsmittel zugefügt wird. Diese Lösungsmittel sind aus dem Stand der Technik (siehe Beschreibungseinleitung) bekannt.

Die Erfindung betrifft weiterhin eine entsprechende Vorrichtung zur Durchführung des Verfahrens. Erfindungsgemäß ist dazu vorgesehen, daß ein evakuierbarer Behälter eingesetzt wird, der über geeignete Ein- und Ausgänge für das Fluidisationsgas und über eine geeignete Zuführung für das flüssige Behandlungsgut verfügt. Der Eingang für das Fluidisationsgas ist dabei bevorzugt so angeordnet, daß das Gas den Behälter von unten nach oben durchströmt. Der Behälter selbst kann dabei beliebig ausgestaltet werden. Vorzugsweise ist der Behälter so ausgelegt, daß er an der Seite, an der das Fluidisationsgas eingeführt wird, konisch zuläuft. Hierbei werden zylindrische Behälter verwendet, die auf der Gaseinleitungsseite verjüngend ausgeführt sind. Durch diese Ausgestaltungsform ist es möglich, daß nach Abschalten des Prozesses sich das feinverteilte Granulat in der unteren konisch zulaufenden Behälterseite sammelt. Der evakuierbare Behälter kann auch mit einer Mikrowelleneinrichtung und/oder mit einem UV-durchlässigen Fenster versehen sein. Bevorzugt wird beim erfindungsgemäßen Verfahren das Fluidisationsgas mittels einer Ringleitung im Kreislauf durch den Behälter geführt. In diesem Falle sind dann in der Ringleitung die entsprechenden Konditionierungseinrichtungen untergebracht.

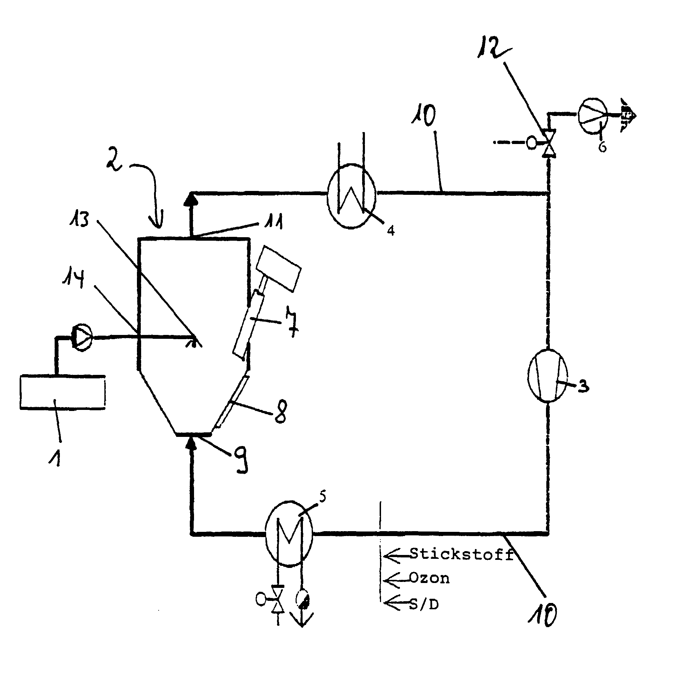

Weitere Einzelheiten, Vorzüge und Vorteile der Erfindung ergeben sich aus dem nachfolgenden Beispiel sowie anhand von Fig. 1.

Fig. 1 zeigt schematisch den Aufbau einer Vorrichtung zur Durchführung des erfindungsgemäßen Verfahrens, wobei hier die bevorzugte Ausführungsform gezeigt ist, bei der das Fluidisationsgas im Kreislauf geführt ist.

Der Behälter 2 ist im Beispielsfall in zylindrischer Form ausgeführt, und auf der Seite, auf der das Fluidisationsgas zugeführt wird, ist er konisch ausgebildet. In die konische Seite des evakuierbaren Behälters 2 führt dabei der Einlaß 9, der im Beispielsfall nach Fig. 1 in Form eines klappbaren Anströmbodens ausgeführt ist. Gleichzeitig ist in der Ummantelung der konischen Seite des Behälters 2 ein Fenster 8 eingebaut, das UV-durchlässig ist. Das Fenster 8 ist so ausgelegt, daß durch eine externe Lichtquelle (nicht abgebildet) das Behandlungsgut noch zusätzlich nachträglich mit UV-Licht bestrahlt werden kann.

Die UV-Lichtintensität kann dabei im Bereich von 1-2 mW/cm².min liegen. Der Behälter 2 verfügt darüber hinaus noch über eine Mikrowelleneinrichtung 7 zur Unterstützung der Trocknung und/oder zur nachfolgenden Wärmebehandlung. Zur Kreislaufführung des Fluidisationsgases ist eine Ringleitung 10 vorgesehen, die vom Ausgang 11 bis zum Einlaß 9 des Behälters 2 führt. In der Ringleitung 10 sind zur Konditionierung ein Kondensator 4 und ein Wärmeaustauscher 5 angeordnet.

Ergänzend kann noch Ozon ebenfalls zur Inaktivierung eingespeist werden. Die Umwälzung des Fluidisationsgases erfolgt durch ein Gebläse 3. Für den Anwendungsfall, das im evakuierbaren Behälter 2 ein reduzierter Betriebsdruck eingestellt werden muß, ist eine entsprechende Vakuumpumpe 6 vorgesehen, die mittels eines Ventils 12 von der Ringleitung 10 abgekoppelt werden kann. Zur Zuführung des flüssigen Behandlungsgutes ist eine Vorlage 1 vorgesehen, aus der das Behandlungsgut über den Einlaß 14 zum Sprühkopf 13 geführt wird.

Im folgenden wird das Verfahren durch ein Beispiel näher erläutert. Das Beispiel betrifft die Trocknung von Human-Blutplasma mit einer Vorrichtung nach Fig. 1.

Frisch aufgetautes Human-Blutplasma wird bei einer Temperatur von ca. 0 bis 4°C über die Düse 13 (Zweistoffdüse 9) in die leere Wirbelschichtkammer 2 eingesprüht. Die Flüssigkeit kann entweder nach dem Top-Spray-Verfahren (siehe Fig. 1) von oben auf das Wirbelbett gesprüht oder - über eine in der Höhe des Anströmbodens angebrachte Sprüheinrichtung - von unten im Gleichstrom mit dem Trägergas in die Wirbelkammer eingesprüht werden (Bottom-Spray-Verfahren).

Die mittlere Fluidisationsgeschwindigkeit beträgt dabei ca. 0,4 bis 0,6 m/s.

Das Anfahren des Prozesses erfolgt vorzugweise nach einem Verfahren gemäß DE 35 16 967 A1. Beim anschließenden eigentlichen Wirbelschichttrocknen wird der Betriebsdruck mit der Pumpe 6 auf ca. 10 KPa abgesenkt. Die Zulufttemperatur liegt bei ca. 35°C. Der Feuchtegehalt der Zuluft oder des Trägergases sollte kleiner 30 % sein. Durch die Druckabsenkung auf einen Betriebsdruck von 10 KPa erniedrigt sich die Siedetemperatur der zu entfernenden Flüssigkeit (z.B. Siedetemperatur Wasser bei 10 KPa beträgt ca. 45°C) sowie die Kühlgrenz- bzw. Gutsoberflächentemperatur (theoretisch ca. 5°C). Dadurch lassen sich niedrige produktschonende Trocknungstemperaturen (gemessene Produkttemperaturen lagen bei ca. 10 bis 15°C) realisieren. In dieser Phase wird die Trocknung mit einer Mikrowellenheizung 7 unterstützt. Die Mikrowelle wird bei einer Frequenz von 2450 MHz und mit einer maximalen Leistung von 1,2 kW eingekoppelt. Die Einstrahlung der Mikrowelle erfolgt produkttemperaturgeregelt, d.h. Zeitpunkt und Dauer der Mikrowelleneinstrahlung sowie die eingestrahlte aktuelle Maximalleistung werden in Abhängigkeit einer festgelegten, maximal zulässigen Produkttemperatur geregelt.

Aufgrund des bekannten Vorteils von Mikrowellenanwendungen, die durch die trägheitslose Energieübertragung eine sehr schnelle Prozeßregelung ermöglicht, ist diese Form der Energiezufuhr geeignet, eine individuelle Prozeßführung bei Trocknungs- und Erwärmungsprozessen zu ermöglichen. Die maximal zulässige Produkttemperatur ist bei 25°C festgelegt. Durch Bestimmung einer entsprechenden Kinetik, die einen formalen Zusammenhang zwischen Temperaturbelastung, Produkt-Wassergehalt und davon abhängiger Wirkstoff aktivitätsminderung herstellt, ist diese exakt bestimmbar. Die bei diesen Bedingungen mögliche Sprührate beträgt bei rein konvektiver Wirbelschichttrocknung ca. 0,9 kg/h. Wird die Trocknungsleistung durch eine produkttemperaturgeregelte Mikrowellenstrahlung ergänzt, kann mit einer Sprührate von ca. 1,9 kg/h getrocknet werden. Als mittlerer Produktwassergehalt in dem fluidisierten Produkt stellt sich ein konstanter Wassergehalt von ca. 10 bis 13 % ein. Die Trocknung erfolgt bis zu einem Produktfeuchtegehalt, bei der der Wirkstoff für eine weitere Behandlung, Lagerung oder Distribution ausreichend stabilisiert vorliegt.

Nach oder gegen Ende der eigentlichen Trocknung erfolgt die Wärmebehandlung zur Inaktivierung von Viren in der gleichen Wirbelkammer 2. Die Fluidisation des getrockneten Produktes sorgt für eine möglichst gleichmäßige und effektive Wärmeübertragung zwischen Trägergas und den einzelnen Partikeln. Die Erwärmung kann z.B. bei einem Druck von 100 KPa und in einer auf 60°C erwärmten Inertgasatmosphäre (z.B. Stickstoff) erfolgen. In Abhängigkeit der gemessenen Produkttemperatur wird die Temperatur der Inertgasatmosphäre so geregelt, daß eine maximale Temperatur von 70°C nicht überschritten wird. Durch die Mikrowellenstrahlung kann die Erwärmung zusätzlich unterstützt werden, mittels der UV-Strahlung ist eine zusätzliche Vireninaktivierung möglich.

Die Dauer der Wärmebehandlung orientiert sich an der zu erreichenden Inaktivierung (Titerreduktion) möglicher Viren. Aufgrund der idealen Wärmeübergangsverhältnisse und der idealen Gleichmäßigkeit der Erwärmung sind deutliche Überhitzungen oder partiell nicht ausreichende Erhitzungen nicht vorhanden, so daß die Erwärmungsdauer von üblicherweise zehn Stunden deutlich unterschritten wird.

## Patentansprüche

1. Verfahren zur Trocknung von humanem Blutplasma, Blutplasmafraktionen oder daraus gewonnenen Blutplasmaprodukten (Behandlungsgut), bei dem das Behandlungsgut im flüssigen oder gelösten Zustand in einen evakuierbaren Behälter gesprüht wird, wobei eine Trocknung mittels eines Fluidisationsgases, in der Wirbelschicht, bis zu feinteiliger Granulatform durchgeführt wird und daß nach dem Ende dieser Trocknung eine zusätzliche Wärmebehandlung, im fluidisierten Zustand, zur Inaktivierung von Viren erfolgt und die Temperatur des Behandlungsgutes während des Trocknungsprozesses gemessen und in Abhängigkeit davon die Erwärmung des Fluidisationsgases eingestellt wird, mit der Maßgabe daß für das jeweilige Behandlungsgut eine möglichst schonende Trocknung herbeigeführt wird.

2. Verfahren nach Anspruch 1, dadurch gekennzeichnet, daß die Temperatur des Fluidisationsgases in Abhängigkeit vom zu behandelnden Gut im Bereich von 15 bis 75°C gehalten wird.

3. Verfahren nach Anspruch 1 oder 2, dadurch gekennzeichnet, daß das Fluidisationsgas im Kreislauf durch den evakuierbaren Behälter geführt wird, wobei im Kreislauf eine Konditionierung mittels eines Kondensators zur Entfeuchtung und eines Wärmeaustauschers zur Erwärmung erfolgt.

4. Verfahren nach mindestens einem der Ansprüche 1 bis 3, dadurch gekennzeichnet, daß als Fluidisationsgas Luft oder ein Inertgas wie Stickstoff eingesetzt wird.

5. Verfahren nach mindestens einem der Ansprüche 1 bis 4, dadurch gekennzeichnet, daß während des Trocknungsprozesses in die Wirbelschicht ein zusätzlicher Energieeintrag erfolgt.

6. Verfahren nach Anspruch 5, dadurch gekennzeichnet, daß in die Wirbelschicht eine Mikrowelle eingekoppelt wird.

7. Verfahren nach einem der Ansprüche 1 bis 6, dadurch gekennzeichnet, daß die zusätzliche Wärmebehandlung mittels einer Mikrowelle erfolgt.

8. Verfahren nach mindestens einem der Ansprüche 1 bis 6, dadurch gekennzeichnet, daß die Inaktivierung der Viren mittels einer UV-Lichtquelle erfolgt.

9. Vorrichtung zur Durchführung des Verfahren nach mindestens einem der Ansprüche 1 bis 8, dadurch gekennzeichnet, daß der evakuierbare Behälter (2) über einen Zugang (14) zum Einsprühen des flüssigen Behandlungsgutes verfügt und daß ein Einlaß (9) für das Fluidisationsgas am evakuierbaren Behälter (2) und ein Ausgang (11) zum Ausleiten des Fluidisationsgases am evakuierbaren Behälter (2) vorgesehen ist und daß am evakuierbaren Behälter (2) eine Mikrowellenvorrichtung (7) so angeordnet ist, daß die Mikrowellenstrahlung in das Wirbelbett einkoppelbar ist.

10. Vorrichtung nach Anspruch 9, dadurch gekennzeichnet, daß eine Ringleitung (10) vorgesehen ist, die den Einlaß (9) und den Ausgang (11) am evakuierbaren Behälter (2) verbindet, wobei in der Ringleitung (10) mindestens ein Kondensator (4), ein Wärmeaustauscher (5) sowie eine Vakuumpumpe (6) angeordnet sind.

11. Vorrichtung nach Anspruch 9 oder 10, dadurch gekennzeichnet, daß der evakuierbare Behälter (2) ein UV-durchlässiges Fenster (8) aufweist, durch das mittels einer externen UV-Lichtquelle UV-Licht in das Wirbelbett einkoppelbar ist.

12. Vorrichtung nach mindestens einem der Ansprüche 9 bis 10, dadurch gekennzeichnet, daß der Einlaß (9) zum Einströmen des Fluidisationsgases ein klappbarer Anströmboden ist.

## Claims

1. Process for drying human blood plasma, blood plasma fractions or blood plasma products obtained therefrom (material to be treated), in which the material to be treated is sprayed into an evacuable container in the liquid or dissolved state, drying being carried out by means of a fluidizing gas, in a fluidized bed, up to finely divided granule form and, after the end of this drying, an additional heat treatment, in the fluidized state, being carried out to inactivate viruses and the temperature of the material to be treated during the drying process being measured and, depending thereon, the warming of the fluidizing gas being adjusted, with the proviso that for the particular material to be treated drying which is as gentle as possible is brought about.

2. Process according to Claim 1, characterized in that the temperature of the fluidizing gas, depending on the material to be treated, is kept in the range from 15 to 75°C.

3. Process according to Claim 1 or 2, characterized in that the fluidizing gas is circulated through the evacuable container, conditioning by means of a condenser for dehumidification and of a heat exchanger for warming taking place in the circulation.

4. Process according to at least one of Claims 1 to 3, characterized in that the fluidizing gas employed is air or an inert gas such as nitrogen.

5. Process according to at least one of Claims 1 to 4, characterized in that during the drying process an additional energy input takes place in the fluidized bed.

6. Process according to Claim 5, characterized in that a microwave is fed into the fluidized bed.

7. Process according to one of Claims 1 to 6, characterized in that the additional heat treatment takes place by means of microwave.

8. Process according to at least one of Claims 1 to 6, characterized in that the inactivation of the viruses takes place by means of a UV light source.

9. Device for carrying out the process according to at least one of Claims 1 to 8, characterized in that the evacuable container (2) has an entrance (14) for spraying in the liquid material to be treated and that an inlet (9) for the fluidizing gas is provided on the evacuable container (2) and an outlet (11) for leading off the fluidizing gas is provided on the evacuable container (2) and that a microwave device (7) is arranged on the evacuable container (2) such that the microwave radiation can be fed into the fluidized bed.

10. Device according to Claim 9, characterized in that a ring line (10) is provided which connects the inlet (9) and the outlet (11) to the evacuable container (2), at least one condenser (4), a heat exchanger (5) and a vacuum pump (6) being arranged in the ring line (10).

11. Device according to Claim 9 or 10, characterized in that the evacuable container (2) has a UV-transparent window (8) through which UV light can be fed into the fluidized bed by means of an external UV light source.

12. Device according to at least one of Claims 9 and 10, characterized in that the inlet (9) for the inflow of the fluidizing gas is a hinged inflow bottom.

## Revendications

1. Procédé pour le séchage de plasma de sang humain, de fractions de plasma de sang ou de produits de plasma de sang obtenus à partir de ceux-ci (produit à traiter), procédé dans lequel le produit à traiter est aspergé, à l'état liquide ou dissous, dans un récipient pouvant être mis sous vide, un séchage étant effectué au moyen d'un gaz de fluidisation dans la couche turbulente jusqu'à obtention d'une forme de granulés fins, et dans lequel, après la fin de ce séchage, un traitement thermique supplémentaire est effectué à l'état fluidisé pour l'inactivation de virus et la température du produit à traiter est mesurée pendant le processus de séchage, et en fonction de celle-ci, le chauffage du gaz de fluidisation est réglé, de manière à effectuer un séchage le plus protecteur possible pour le produit à traiter considéré.

2. Procédé selon la revendication 1, caractérisé en ce que la température du gaz de fluidisation est maintenue à l'intérieur de la gamme de 15 à 75°C, en fonction du produit devant être traité.

3. Procédé selon la revendication 1 ou 2, caractérisé en ce que le gaz de fluidisation est amené à travers le récipient pouvant être évacué dans un circuit, un conditionnement étant effectué dans le circuit au moyen d'un condenseur pour la déshumidification et d'un échangeur de chaleur pour le chauffage.

4. Procédé selon au moins une des revendications 1 à 3, caractérisé en ce que de l'air ou un gaz inerte, comme de l'azote, est utilisé en tant que gaz de fluidisation.

5. Procédé selon au moins une des revendications 1 à 4, caractérisé en ce qu'un apport d'énergie supplémentaire dans la couche turbulente est effectué pendant le processus de séchage.

6. Procédé selon la revendication 5, caractérisé en ce qu'un micro-onde est couplé à la couche turbulente.

7. Procédé selon l'une quelconque des revendications 1 à 6, caractérisé en ce que le traitement thermique supplémentaire est effectué au moyen d'un micro-onde.

8. Procédé selon l'une au moins des revendications 1 à 6, caractérisé en ce que l'inactivation des virus est effectuée au moyen d'une source de lumière ultraviolette.

9. Dispositif pour la mise en oeuvre du procédé selon l'une au moins des revendications 1 à 8, caractérisé en ce que le récipient (2) pouvant être mis sous vide dispose d'un accès (14) pour l'injection par aspersion du produit à traiter liquide, et en ce qu'il est prévu une entrée (9) pour le gaz de fluidisation sur le récipient (2) pouvant être mis sous vide et une sortie (11) pour l'évacuation du gaz de fluidisation sur le récipient pouvant être mis sous vide (2), et en ce qu'un dispositif à micro-ondes (7) est disposé sur le récipient pouvant être mis sous vide (2) de façon telle que le rayonnement micro-onde peut être couplé au lit turbulent.

10. Dispositif selon la revendication 9, caractérisé en ce qu'il est prévu une conduite (10) en circuit fermé est prévue qui relie l'entrée (9) et la sortie (11) disposées sur le récipient (2) pouvant être mis sous vide, au moins un condenseur (4), un échangeur de chaleur (5) et une pompe à vide (6) étant disposés dans la conduite (10) en circuit fermé.

11. Dispositif selon la revendication 9 ou 10, caractérisé en ce que le récipient (2) pouvant être mis sous vide présente une fenêtre (8) transparente aux rayons ultraviolets par laquelle, au moyen d'une source de lumière ultra-violette externe, de la lumière ultra-violette peut être couplée au lit turbulent.

12. Dispositif selon l'une au moins des revendications 9 à 10, caractérisé en ce que l'entrée (9) pour l'admission du gaz de fluidisation est un clapet d'insufflation basculant.
